# EUROPEAN PATENT APPLICATION

(11) **EP 3 425 056 A1**
(43) Date of publication of application: **09.01.2019**
(21) Application number: 17305892.6
(22) Date of filing: 07.07.2017
(51) Int. Cl.: C12Q 1/68

(54) **METHOD FOR PROGNOSING FIBROSIS PROGRESSION**

(71) Applicant: Genepred Biotechnologies, 13006 Marseille (FR); INSERM (Institut National de la Santé et de la Recherche Médicale), 75013 Paris (FR); Université d'Aix Marseille, 13007 Marseille (FR)
(72) Inventor: DESSEIN, Alain, 13009 Marseille (FR); SERTORIO, Mathieu, CT 45239 Cincinnati (US); ARGIRO, Laurent, 13385 Marseille (FR)
(74) Representative: Cabinet Plasseraud

(57) **Abstract**

The present invention relates to a method for detecting a predisposition to severe fibrosis and/or prognosing fibrosis progression to a severe stage of a subject in need thereof, comprising detecting at least one single nucleotide polymorphism (SNP) in SMAD3 gene and at least one SNP in BMP7 gene, in a biological sample of said subject, wherein the presence of at least one SNP in SMAD3 gene and at least one SNP in BMP7 gene are indicative of a predisposition to severe fibrosis or to progress to severe fibrosis.

## Description

### FIELD OF THE INVENTION

The invention relates to a method for detecting a predisposition to and/or prognosing fibrosis progression of a subject in need thereof, comprising the detection of at least one single nucleotide polymorphism (SNP) in SMAD3 gene and of at least one SNP in BMP7 gene, in a biological sample of said subject.

### BACKGROUND OF THE INVENTION

Chronic liver inflammation caused by schistosome eggs, hepatitis C or B virus, toxic substances such as metabolites of alcohol or derivatives of fat, stimulate the deposition of extracellular matrix proteins (ECMP) in damaged tissues that form a dense network of fibrils referred to as fibrosis tissue.
In most patients ECMP depots are turned over and replaced by recently divided cells. In others that may represent up to 25% of the affected population, fibrosis accumulates, increases intrahepatic resistance to blood flow, promotes angiogenesis and causes a profound alteration of hepatic vascularization and the development of gastric varices. Bleeding from these varices and loss of hepatic architecture aggravate tissue necrosis, fibrosis and may cause liver failure and death. Severe fibrosis may also leads to hepatocellular carcinoma.

Current diagnosis of hepatic fibrosis is mostly based on liver biopsy, elastometry and ultrasound analysis.
Liver biopsy is an invasive and costly procedure, and samples only a small portion of the liver. Thus it cannot afford a global assessment of hepatic fibrosis, and is subject to sampling variation and inter- and intra-observer error. In addition, it includes potential complications such as local hematoma, infection, pain related to the biopsy and death in less than 1% of the biopsied subjects.
Noninvasive tests such as elastography or endoscopy are also used.
Fibroscan is an approach to staging hepatic fibrosis, which is based on elastography and provides rapid measurement of mean hepatic tissue stiffness (Ziol et al, 2005). A probe is employed to transmit a vibration of low frequency and amplitude into the liver. This vibration wave triggers an elastic shear wave, whose velocity through the liver is directly proportional to tissue stiffness measured in kilopascals (kPa). It also measures the ultrasound attenuation in decibel per meter (dB/m). Fibroscan allows determining the presence and stage of hepatic fibrosis. However, the limitations of this technique are associated with attenuation of elastic waves in fluid or adipose tissue, which would impair assessment of fibrosis in patients. In addition, it is an extremely expensive instrument.

Nowadays, genetic prognosis and diagnosis of hepatic fibrosis is being studied.
The great majority of severe cases are observed in a limited number of families and fibrosis was shown to be dependent on the effects of a major genetic locus where mutations in two genes, CTGF and IL22RA2, have been identified as aggravating liver fibrosis. The effects of these genetic variants were described first in Chinese infected by schistosomes and then the results were extended to populations of Africa and South America. Moreover, the inventors made the hypothesis that the same loci were also controlling severe fibrosis of other etiologic causes and demonstrated that cirrhosis caused by HCV is indeed strongly dependent on mutations in IL22RA2, as shown in WO2013/020904. On the other hand, WO2010/094740 identifies CTGF (CCN2) as a fibrosis susceptibility gene.
Although the already reported genetic associations were highly significant, the relative risk associated with these variants is modest. As a consequence, the fraction of heritability that was explained by the observed variants remains modest and most of these variants account for less than 2% of the phenotypic variance.

There is thus a need for an efficient and reliable method for prognosing fibrosis progression of a given subject over time, which may be noninvasive, easy and quick to perform. Such a prognosis method would have to be sensitive and specific, so as to determine the outcome of the fibrosis. There is also a need for an efficient method for detecting a predisposition to fibrosis progression in a subject.

There is also a need for a method for selecting fibrosis patients in order to target treatment for these subjects. This will increase compliance to treatment, and reduce the cost of mass treatment programs.

### SUMMARY OF THE INVENTION

The inventors have surprisingly discovered that several polymorphisms in BMP7 and in SMAD3 genes aggravate hepatic fibrosis, especially on a cohort infected with *Schistosoma mansoni,* and that SMAD3 genetic variants have more pronounced effects on hepatic fibrosis when the effects of BMP7 variants are taken into account. Interestingly, as shown in the examples, combinations of both variant types accounted for 12% of phenotypic variance. Thus, this is a clear demonstration that certain polymorphisms in SMAD3 and BMP7 genes are associated with fibrosis aggravation in any tissues. These variants may then be used in a method for an efficient prognosis of fibrosis progression.

The invention provides a reliable method for predicting fibrosis progression, especially hepatic fibrosis. Said method allows detecting a predisposition to fibrosis progression.

Thus, the invention provides a method for detecting a predisposition to and/or prognosing fibrosis progression of a subject in need thereof, comprising detecting at least one single nucleotide polymorphism (SNP) in SMAD3 gene and at least one SNP in BMP7 gene, in a biological sample of said subject,
wherein the presence of at least one SNP in SMAD3 gene and at least one SNP in BMP7 gene are indicative of a predisposition to fibrosis or of fibrosis progression.

### DETAILED DESCRIPTION OF THE INVENTION

The present invention relates to a method for detecting a predisposition to and/or prognosing fibrosis progression of a subject in need thereof, comprising detecting at least one single nucleotide polymorphism (SNP) in SMAD3 gene and at least one SNP in BMP7 gene, in a biological sample of said subject, wherein the presence of at least one SNP in SMAD3 gene and at least one SNP in BMP7 gene are indicative of a predisposition to fibrosis or of fibrosis progression.

The invention also relates to a method for detecting a predisposition and/or prognosing fibrosis progression of a subject in need thereof, comprising detecting, in a biological sample of said subject:
a) the presence of at least one of the following genotypes in SMAD3 gene locus:
   - genotype TT with respect to SNP rs1470001,
   - genotype CC or CT with respect to SNP rs 10152544,
   - genotype GT with respect to SNP rs11071937, and
   - genotype CC with respect to SNP rs11632964, and
b) the presence of at least one of the following genotypes in BMP7 gene locus:
   - genotype CC or TT with respect to SNP rs162314,
   - genotype TT or CT with respect to SNP rs6127988, and
   - genotype CC with respect to SNP rs7352741,
wherein a) the presence of at least one SNP in SMAD3 gene locus and b) the presence of at least one SNP in BMP7 gene locus are indicative of a predisposition to fibrosis or of fibrosis progression.

Preferably the method comprises genotyping at least one SNP in SMAD3 gene, selected in the group consisting of rs1470001, rs10152544, rs11071937 and rs11632964.
In an embodiment, the presence of a T allele with respect to SNP rs1470001, more particularly of a TT genotype, is deleterious for the patient, i.e. it is indicative of a patient being likely to develop abnormal deposit of ECMP, or fibrosis, especially hepatic fibrosis.
In an embodiment, the presence of a C allele with respect to SNP rs10152544, more particularly of a CC or CT genotype, is deleterious for the patient, i.e. it is indicative of a patient being likely to develop abnormal deposit of ECMP, or fibrosis, especially hepatic fibrosis.
In an embodiment, the presence of a GT genotype with respect to SNP rs11071937 is deleterious for the patient, i.e. it is indicative of a patient being likely to develop abnormal deposit of ECMP, or fibrosis, especially hepatic fibrosis.
In an embodiment, the presence of a C allele with respect to SNP rs11632964, more particularly of a CC genotype, is deleterious for the patient, i.e. it is indicative of a patient being likely to develop abnormal deposit of ECMP, or fibrosis, especially hepatic fibrosis.

Preferably the method comprises genotyping at least one SNP in BMP7 gene, selected in the group consisting of rs162314, rs6127988 and rs7352741.
In an embodiment, the presence of a CC or TT genotype with respect to SNP rs162314, is deleterious for the patient, i.e. it is indicative of a patient being likely to develop abnormal deposit of ECMP, or fibrosis, especially hepatic fibrosis.
In an embodiment, the presence of a T allele with respect to SNP rs6127988, more particularly of a TT or CT genotype, is deleterious for the patient, i.e. it is indicative of a patient being likely to develop abnormal deposit of ECMP, or fibrosis, especially hepatic fibrosis.
In an embodiment, the presence of a C allele with respect to SNP rs7352741, more particularly of a CC genotype, is deleterious for the patient, i.e. it is indicative of a patient being likely to develop abnormal deposit of ECMP, or fibrosis, especially hepatic fibrosis. By **"SNP"** or **"single nucleotide polymorphism",** it is meant a single nucleotide variation in a genetic sequence that occurs at appreciable frequency in the population. There are millions of SNPs in the human genome. Most commonly, these variations are found in the DNA between genes. When SNPs occur within a gene or in a regulatory region near a gene, they may play a more direct role in disease by affecting the gene's function.
Linkage disequilibrium (LD) is defined as the non-random association of alleles at different loci across the genome. Alleles at two or more loci are in LD if their combination occurs more or less frequently than expected by chance in the population. When there is a causal locus in a DNA region, due to LD, one or more SNPs nearby are likely associated with the trait too. Therefore, any SNPs in strong LD (yielding a r²>0.04) with a given SNP associated with an abnormal ECMP deposit will be associated with this trait.
Identification of additional SNPs in linkage disequilibrium with a given SNP involves: (a) amplifying a fragment from the genomic region comprising or surrounding a first SNP from a plurality of individuals; (b) identifying second SNPs in the genomic region harboring or surrounding said first SNP; (c) conducting a linkage disequilibrium analysis between said first SNP and second SNPs; and (d) selecting said second SNPs as being in linkage disequilibrium with said first marker. Sub-combinations comprising steps (b) and (c) are also contemplated. Methods to identify SNPs and to conduct linkage disequilibrium analysis can be carried out by the skilled person without undue experimentation by using well-known methods.
Other SNPs showing strong LD with the SNPs of the present invention, will also be associated with fibrosis. They may be used as additional markers as well.

The method of the invention comprises detecting at least one SNP in SMAD3 gene, preferably selected from rs1470001, rs10152544, rs11071937 and rs1632964.
Said SNPs are summarized in the Tables 1A below:

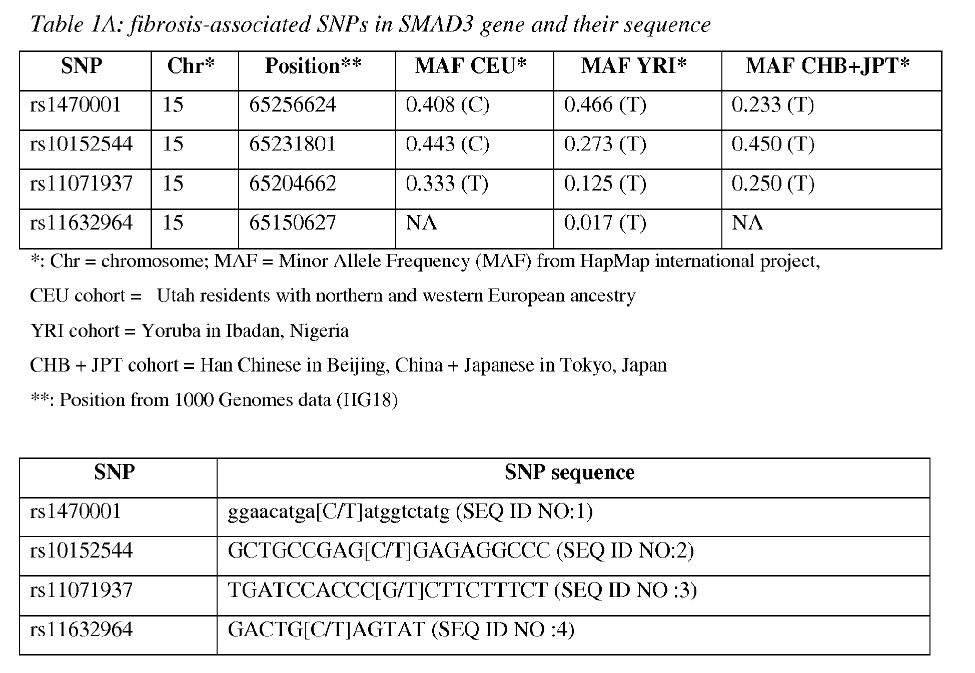

The method of the invention also comprises detecting at least one SNP in BMP7 gene, preferably selected from rs162314, rs6127988 and rs7352741.
Said SNPs are summarized in the Tables 1B below:

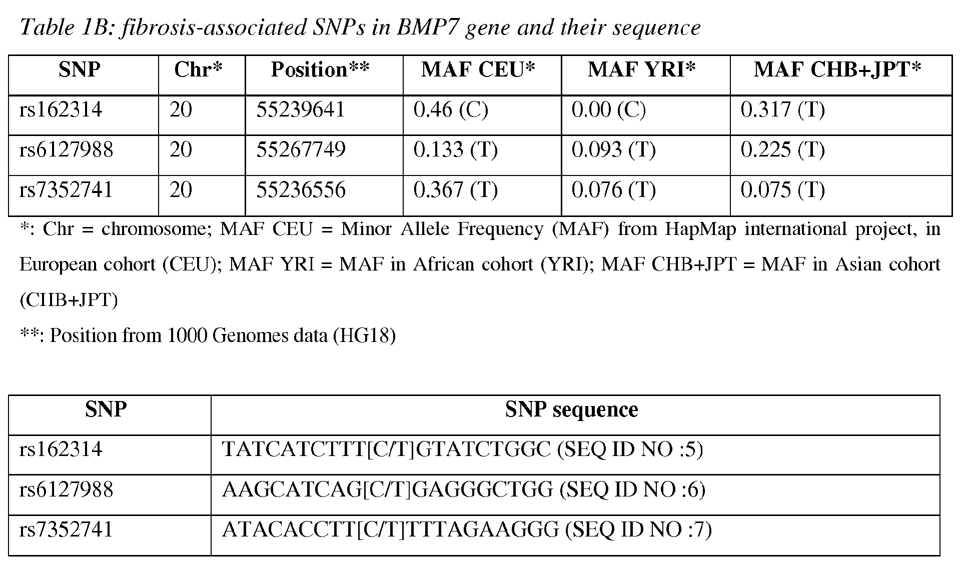

The term **"subject"** as used herein refers to a mammalian, preferably a human. Preferably, the subject may be infected with a virus, preferably a virus selected from the Flaviviridae, such as Hepatitis A or C or B Virus. According to another embodiment, preferably, the subject may be infected by Schistosoma, such as *Schistosoma mansoni, Schistosoma haematobium, Schistosoma japonicum, Schistosoma mekongi* or *Schistosoma intercalatum.* These schistosoma are responsible for bilharzia (or schistosomiasis). According to another embodiment, preferably, the subject has a diet which is fat-rich, and/or alcohol-rich. Preferably, in such cases, the subject is afflicted by fatty liver, overweight, obesity and/or alcoholism.

Within the context of this invention, **"fibrosis"** designates all types of human fibrosis, i.e. abnormal ECMP depot, occurring in a human fibrotic disease. Such a human fibrotic disease may be chosen from hepatic fibrosis (notably of various grades, including cirrhosis), cutaneous keloid, hypertrophic scars, scleroderma, obesity and alcoholism. Preferably, fibrosis is chosen from:
- hepatic fibrosis, comprising hepatic fibrosis of various grades including cirrhosis, alcoholic and non-alcoholic,
- fibrosis of the skin tissue, particularly due to cutaneous keloid, hypertrophic scars or scleroderma,
- fibrosis of the adipose tissue, such as fibrosis due to obesity (such as non-alcoholic fatty liver disease (NAFLD) or non-alcoholic steatohepatitis (NASH)),
- pulmonary fibrosis and
- kidney fibrosis.

Within the context of this invention, **"hepatic fibrosis"** or "HF" designates all types of fibrosis occurring in a liver, tissue thereof or any part of tissue thereof. Hepatic fibrosis occurs especially in response to an injury. Hepatic fibrosis can be the common response to chronic liver injury, ultimately leading to cirrhosis and its complications, portal hypertension, liver failure, and hepatocellular carcinoma. Various types of chronic liver injury can cause hepatic fibrosis, such as:
- hepatic fibrosis due to a chemical agent (such as an hepato-toxic drug or alcohol),
- hepatic fibrosis due to fat (such as NAFLD or NASH),
- hepatic fibrosis due to a bacterial infection (such as brucellosis),
- hepatic fibrosis due to a parasitic infection (such as bilharzia), or
- hepatic fibrosis due to viral infections (such as hepatic A virus (HAV), hepatic B virus (HBC) or hepatic C virus (HCV) infections).

**"Fibrosis of the skin tissue"** may be chosen from cutaneous keloid, hypertrophic scars and scleroderma.
A cutaneous keloid is an excessive growth of scar tissue on the skin. A hypertrophic scar is a cutaneous condition characterized by excessive collagen depot which gives rise to a raised scar, and which, contrary to keloid, does not grow beyond the boundaries of the original wound.
Keloids and hypertrophic scars often occur following trauma, inflammation, surgery, burns and sometimes spontaneously. These disorders represent aberrations in the fundamental processes of wound healing.

Within the context of the present invention, the term **"prognosis"** includes the detection, monitoring, dosing and/or comparison, at various stages, including early, pre-symptomatic stages, and late stages, in adults, children and pre-birth. Prognosis typically includes the prediction of the progression of fibrosis and the characterization of a subject to define the most appropriate treatment.

The **biological sample** of the monitoring method of the invention is isolated from a subject and can include, by way of example and not limitation, bodily fluids and/or tissue extracts such as homogenates or solubilized tissue obtained from said subject. Bodily fluids useful in the present invention include blood, urine, saliva or any other bodily secretion or derivative thereof. As used herein "blood" includes whole blood, plasma, serum, circulating epithelial cells, constituents, or any derivative of blood.
Preferably the biological sample used in the invention is a biopsy, a blood sample, a saliva sample or a urine sample, more preferably a biopsy or a blood sample. The blood sample may be a freshly isolated blood sample (<48h) or a blood sample which has been obtained previously and kept frozen until use.

The detections of at least one SNP in SMAD3 gene and of at least one SNP in BMP7 gene may independently be performed by selective hybridization assay, sequencing assay or microsequencing assay. Other suitable methods include allele-specific oligonucleotide (ASO), allele-specific amplification, single-stranded conformation analysis (SSCA), fluorescent *in situ* hybridization (FISH), gel migration, clamped denaturing gel electrophoresis, heteroduplex analysis or chemical mismatch cleavage. Some of these approaches (such as SSCA) are based on a change in electrophoretic mobility of the nucleic acids, as a result of the presence of an altered sequence. According to these techniques, the altered sequence is visualized by a shift in mobility on gels. The fragments may then be sequenced to confirm the alteration. Some others are based on specific hybridization between nucleic acids from the subject and a probe specific for wild-type or altered SMAD3 or BMP7 gene. The probe may be in suspension or immobilized on a substrate. The probe is typically labelled to facilitate detection of hybrids.

The present invention also relates to a kit for detecting a predisposition and/or prognosing fibrosis progression of a subject.
Said kit allows detecting at least one SNP in SMAD3 gene and at least one SNP in BMP7 gene, in a biological sample. Thus, according to the invention, said kit comprises at least the following sequences SEQ ID NO:8 to SEQ ID NO:14:
SMAD3 SNP assays:

| **SNP** | **TaqMan Assay Id** | **Context sequence [VIC/FAM]** |
|---|---|---|
| rs1470001 | custom design | TAGACAAGACAAAAAGGGAACATGA[C/T]ATGGTCTATGGCTCTGGTTGGTGGA (SEQ ID NO:8) |
| rs10152544 | C___2112954_10 | CTACATGAACAGGCCTGCTGCCGAG[C/T]GAGAGGCCCAGATCACCCAGAATGA (SEQ ID NO:9) |
| rs11071937 | C___2112999_10 | GGCAGCTCTGGTTGCTGATCCACCC[G/T]CTTCTTTCTTTATAGAGATTAAGAG (SEQ ID NO:10) |
| rs11632964 | C___2720852_10 | TATCCAAAGGAAGCTTTTTGGACTG[C/T]AGTATGATGTTGGGAACTTTTTGGT (SEQ ID NO:11) |

BMP7 SNP assays:

| **SNP** | **TaqMan Assay Id** | **Context sequence [VIC/FAM]** |
|---|---|---|
| rs162314 | C___1364063_10 | GGGCCTCATACACTATATCATCTTT[C/T]GTATCTGGCTTCTTCCATTTCACAG (SEQ ID NO:12) |
| rs6127988 | custom design | TTGGCCAGGCAGCCCCAAGCATCAG[C/T]GAGGGCTGGAAGCCTTCACTGTGAA (SEQ ID NO:13) |
| rs7352741 | C___1364054_20 | GGAAGTGACATACACCATACACCTT[C/T]TTTAGAAGGGATAATCTGGTACAGG (SEQ ID NO:14) |

Finally, the present invention also relates to a method for treating fibrosis in a subject in need thereof, comprising the following steps:
i) detecting at least one single nucleotide polymorphism (SNP) in SMAD3 gene and at least one SNP in BMP7 gene, in a biological sample of said subject,
   wherein if at least one SNP in SMAD3 gene and at least one SNP in BMP7 gene are detected, then said subject is at risk of developing a severe fibrosis; and
ii) subsequently treating said subject with a treatment.

Within the context of this invention, subject is at risk of developing a severe fibrosis refers to the phenotype of a subject afflicted by a severe fibrosis or at risk of developing a severe form of fibrosis. Therefore the identified subject will be treated by a treatment so that the viral load is decreased, or at least one of his symptoms is alleviated, or the development of fibrosis is stopped, or slowed down.
The treatment of step ii) above may be chosen from antiviral drugs (antiviral agent and/or interferons).

This method of the invention allows an efficient prognosis of fibrosis progression, and thus, a better treatment. Indeed, as there is no treatment for curing severe fibrosis cases, the method of the invention allows an earlier prognosis of severe fibrosis cases, increasing the chance of a better outcome.

The invention will be further described by the following examples, which are not intended to limit the scope of the protection defined by the claims.

### FIGURE LEGENDS

**Figure 1A** **and** **1B****.** Regional linkage association plot for rs162314 in BMP7 from 1000 Genomes data in CEU population (Utah residents with Northern and Western European Ancestry), R²>0.6, figures in order of appearance: 500kb and 100kb around SNP of interest.
**Figure 2****.** Regional linkage association plot for rs162314 in BMP7 from HapMap3 data in CEU population (Utah residents with Northern and Western European Ancestry), R²>0.6, figures in order of appearance: 500kb around SNP of interest.
**Figure 3A** **and** **3B****.** Regional linkage association plot for rs6127988 in BMP7 from 1000 Genomes data in CEU population (Utah residents with Northern and Western European Ancestry), R²>0.6, figures in order of appearance: 500kb and 100kb around SNP of interest.
**Figure 4****.** Regional linkage association plot for rs7352741 in BMP7 from 1000 Genomes data in CEU population (Utah residents with Northern and Western European Ancestry), R²>0.6, 500kb around SNP of interest.
**Figure 5****.** Regional linkage association plot for rs1470001 in SMAD3 from 1000 Genomes data in CEU population (Utah residents with Northern and Western European Ancestry), R²>0.6, 500kb around SNP of interest.
**Figure 6****.** Regional linkage association plot for rs10152544 in SMAD3 from 1000 Genomes data in CEU population (Utah residents with Northern and Western European Ancestry), R²>0.6, 500kb around SNP of interest.
**Figure 7****.** Regional linkage association plot for rs10152544 in SMAD3 from HapMap release 22 data in CEU population (Utah residents with Northern and Western European Ancestry), R²>0.6, 500kb around SNP of interest.
**Figure 8****.** Regional linkage association plot for rs11071937 in SMAD3 from 1000 Genomes data in CEU population (Utah residents with Northern and Western European Ancestry), R²>0.6, 500kb around SNP of interest.
**Figure 9****.** Regional linkage association plot for rs11071937 in SMAD3 from HapMap release 22 data in CEU population (Utah residents with Northern and Western European Ancestry), R²>0.6, 500kb around SNP of interest.
**Figure 10****.** Regional linkage association plot for rs11632964 in SMAD3 from 1000 Genomes data in CEU population (Utah residents with Northern and Western European Ancestry), R²>0.6, 500kb around SNP of interest.
**Figure 11****.** Regional linkage association plot for rs11632964 in SMAD3 from HapMap3 data in CEU population (Utah residents with Northern and Western European Ancestry), R²>0.6, 500kb around SNP of interest.

### Example 1: Several variants in BMP7 and SMAD3 aggravate hepatic fibrosis in schistosome infected subjects

### Materials and methods

### Study population and evaluation of hepatic fibrosis

The human protocols were approved by the research ethics committees of the University do Triangulo Mineiro (Uberaba, Brazil) and University Estadual de Pernambucco (Recife). The protocols were also approved by French INSERM ethics committees and by the CNIL. Subjects infected with schistosomes were obtained from populations exposed to infection with *S. mansoni* in the state of Pernambucco.

### Evaluation of hepatic fibrosis

Fibrosis in individuals infected with schistosomes was evaluated with Ultrasound (US) that was carried out with a portable ultrasound machine and a 3.5MHz convex probe (LOGIQ Book XP), based on the standardized procedures of the World Health Organization (WHO) (Parasitol Res. 2014 Nov;113(11):3915-25. doi: 10.1007/s00436-014-4117-0. Epub 2014 Sep 27. The WHO ultrasonography protocol for assessing hepatic morbidity due to Schistosoma mansoni. Acceptance and evolution over 12 years. el Scheich T¹, Holtfreter MC, Ekamp H, Singh DD, Mota R, Hatz C, Richter J), in individuals infected with *S. mansoni* manifests as central fibrosis (CentF) around the central vein.

WHO guidelines graded fibrosis as A, B, C, D, E, or F. The A fibrosis pattern is a normal liver; diffuse echogenic foci of fibrosis or "starry sky" is graded B; thickness (due to fibrosis) of the venous wall is graded C if discontinuous or CL if continuous; a patch on the central portal vein is graded as D, if the patches are in the parenchyma, fibrosis is graded E and F if these patches extend to the periphery of the organ. Controls were individuals who exhibited no (A) or light fibrosis (grade B or C). The advanced and severe hepatic fibrosis (HF) cases exhibited E, and F grades. Grade D were not included in the study unless associated with a strong CL pattern or with the presence of collaterals or with the presence of oesophageal varices.

### DNA extraction

Genomic DNA was extracted from 2 ml of whole blood with the QIAamp DNA Blood Midi Kit (Qiagen), according to the manufacturer's instructions, and was stored at -20°C until use. DNA concentration and purity were determined by UV spectrophotometric measurements.

### Tag-single nucleotide polymorphism (tag-SNP) selection

Given the lack of published information on Brazilian population, the inventors carried out tag-SNP selection using both cohort of African Yoruba living in Ibadan, Nigeria (YRI) and cohort of Utah residents with ancestry from northern and western Europe (CEU) from the 1000 Genomes project (18), since the study populations were an admixture of Africans, Europeans and local Amerindians. Only SNPs with a minor-allele frequency (MAF) ≥ 5% in at least one of the reference panels were included in the analysis. An optimal set of markers covering all genes (including an extra 10 kb at each end) was selected on the basis of the squared coefficients of correlation (r2 ≥ 0.8) between SNPs, using PLINK software (19).

### Polymorphism genotyping and quality control

TaqMan genotyping: SNPs were genotyped using validated TaqMan probe assays (Applied Biosystems):
SMAD3 SNP assays :

| **SNP** | **TaqMan Assay Id** | **Context sequence [VIC/FAM]** |
|---|---|---|
| rs1470001 | custom design | TAGACAAGACAAAAAGGGAACATGA[C/T]ATGGTCTATGGCTCTGGTTGGTGGA (SEQ ID NO:8) |
| rs10152544 | C___2112954_10 | CTACATGAACAGGCCTGCTGCCGAG[C/T]GAGAGGCCCAGATCACCCAGAATGA (SEQ ID NO:9) |
| rs11071937 | C___2112999_10 | GGCAGCTCTGGTTGCTGATCCACCC[G/T]CTTCTTTCTTTATAGAGATTAAGAG (SEQ ID NO:10) |
| rs11632964 | C___2720852_10 | TATCCAAAGGAAGCTTTTTGGACTG[C/T]AGTATGATGTTGGGAACTTTTTGGT (SEQ ID NO:11) |

BMP7 SNP assays :

| **SNP** | **TaqMan Assay Id** | **Context sequence [VIC/FAM]** |
|---|---|---|
| rs162314 | C___1364063_10 | GGGCCTCATACACTATATCATCTTT[C/T]GTATCTGGCTTCTTCCATTTCACAG (SEQ ID NO:12) |
| rs6127988 | custom design | TTGGCCAGGCAGCCCCAAGCATCAG[C/T]GAGGGCTGGAAGCCTTCACTGTGAA (SEQ ID NO:13) |
| rs7352741 | C___1364054_20 | GGAAGTGACATACACCATACACCTT[C/T]TTTAGAAGGGATAATCTGGTACAGG (SEQ ID NO:14) |

In brief, each reaction contained 12.5 ng of genomic DNA, 900 nM of each primer, 200 nM of each fluorescently labeled probe and TaqMan Universal PCR Master Mix (Applied Biosystems), in a total volume of 5 µl. PCR was conducted under the following conditions: 50°C for 2 minutes, 95°C for 10 minutes and 40 cycles of amplification (95°C for 15 seconds and 60°C for 1 minute). Allele discrimination was achieved with the 7900HT Fast Real-Time PCR System (Applied Biosystems). Quality control criteria similar to those applied in the discovery study were used following TaqMan genotyping. Post genotyping quality control was carried out before testing for association. All procedures were conducted automatically with PLINK software. SNPs displaying significant deviation from Hardy-Weinberg equilibrium (p < 0.01; based on founders only) were excluded from the analysis.

Genotyping was performed on a cohort of 656 subjects (307 with severe hepatic fibrosis, 349 with no or mild hepatic fibrosis) who have been living for more than 25 years in regions endemic for *S.mansoni.* The inventors present below the data of the SNPs that were genotyped with a call rate > 95%.

### Statistical analysis

Group comparisons performed on data were carried out with non-parametric Mann-Whitney tests with SPSS software. Multivariate logistic regression (SPSS statistical software) was used to test the independency of the associations. All covariates were treated as binary covariates as previously described Variants of CTGF are associated with hepatic fibrosis in Chinese, Sudanese, and Brazilians infected with schistosomes. (Dessein A, Chevillard C, Arnaud V, Hou X, Hamdoun AA, Dessein H, He H, Abdelmaboud SA, Luo X, Li J, Varoquaux A, Mergani A, Abdelwahed M, Zhou J, Monis A, Pitta MG, Gasmelseed N, Cabantous S, Zhao Y, Prata A, Brandt C, Elwali NE, Argiro L, Li Y. J Exp Med. 2009 Oct 26;206(11):2321-8. doi: 10.1084/jem.20090383. Epub 2009 Oct 12).

### Results

The inventors have selected 16 tag-SNPs in SMAD3 and genotyped them in the cohort. They have obtained significant (p ≤ 0.01) or suggestive (0.01 <p < 0.1) associations of n (6) SNPs with HF. Logistic regression indicated that certain associations were not independent, these are not reported below.

The upper part of Tables 1 and 2 below shows the results of the univariate testing (each SNP tested separately), the lower part of the tables report the multivariate testing (all SNPs that show a significant or suggestive associations in univariate testing are tested in the model, beginning with the SNPs that showed the strongest associations in the univariate model).

The linkage disequilibrium (LD) is calculated using Haploview 4.0 based on phased genotype data from the International HapMap Project and the 1000 Genomes Project.

### Two SNPs in SMAD3 are independently associated with severe HF

Table 1 (upper part) below shows that 2 SNPs (rs11071937, rs11632964) in SMAD3 showed a significant association with HF, and 2 SNPs (rs1470001 and rs10152544) showed suggestive associations. ODD ratios ranged between 1.4 and 1.65. Each SNP accounted for less than 0.02 of phenotypic variance.

**Table 1. SMAD3 Genotypes associated with advanced hepatic fibrosis in subjects infected with S. mansoni**

| **SNP** | **Aggravating genotypes** | **% in controls¹** | **% in cases¹** | **p** | **OR** | **CI** | **r²** |
|---|---|---|---|---|---|---|---|
| **Univariate analysis** | | | | | | | |
| rs1470001 | TT | 28.5 | 36.0 | 0.02 | 1.47 | (1.05 - 2.06) | 0.011 |
| rs10152544 | CC,CT | 81.3 | 86.6 | 0.08 | 1.48 | (0.96 - 2.26) | 0.007 |
| rs11071937 | GT | 31.0 | 40.9 | 2.10⁻³ | 1.7 | (1.22 - 2.34) | 0.021 |
| rs11632964 | CC | 38.5 | 49.8 | 2.10⁻³ | 1.64 | (1.2 - 2.3) | 0.019 |

| **Multivariate analysis** | | | | | | | |
|---|---|---|---|---|---|---|---|
| rs11071937 | | | | 10⁻³ | 1.7 | (1.24 - 2.4) | 0.04 |
| rs11632964 | | | | 2.10⁻³ | 1.66 | (1.2 - 2.3) | |

| | | | | | | | |
|---|---|---|---|---|---|---|---|
| ¹ Percentage of subjects with the aggravating genotypes in 349 Controls and 307 Cases. Controls are subjects who have been exposed to infections for > 20 years and show no (grade A) or mild (grade B or C) hepatic fibrosis. Cases are subjects with grade E or F Hepatic Fibrosis r² = (r Nagelkerke)² | | | | | | | |

Multivariate analysis presented in the lower part of Table 1 showed that 2 SNPs, rs11071937 (p=10⁻³, OR= 1.7) and rs11632964 (2x10⁻³, 1.66), were independently associated to HF. The regression model accounted for 4% of the phenotypic variance (r²=0.04). SNPs rs10152544 (0.06, 1.5) and rs 1470001 (0.03, 1.5) showed suggestive associations when they were forced in the model.

### At least 3 (and likely four) causal SNPs located in SMAD3 regulate hepatic fibrosis

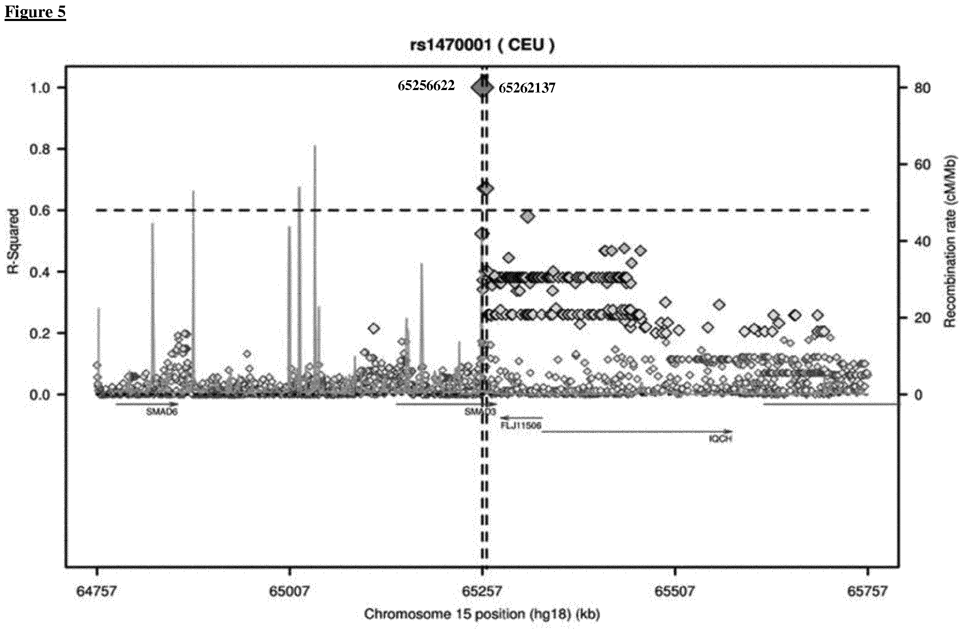

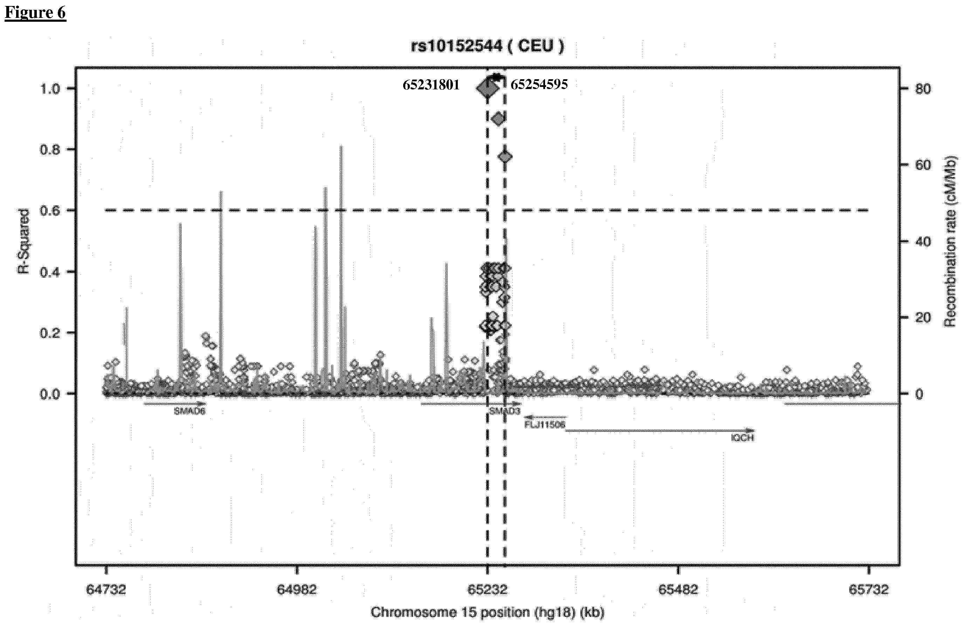

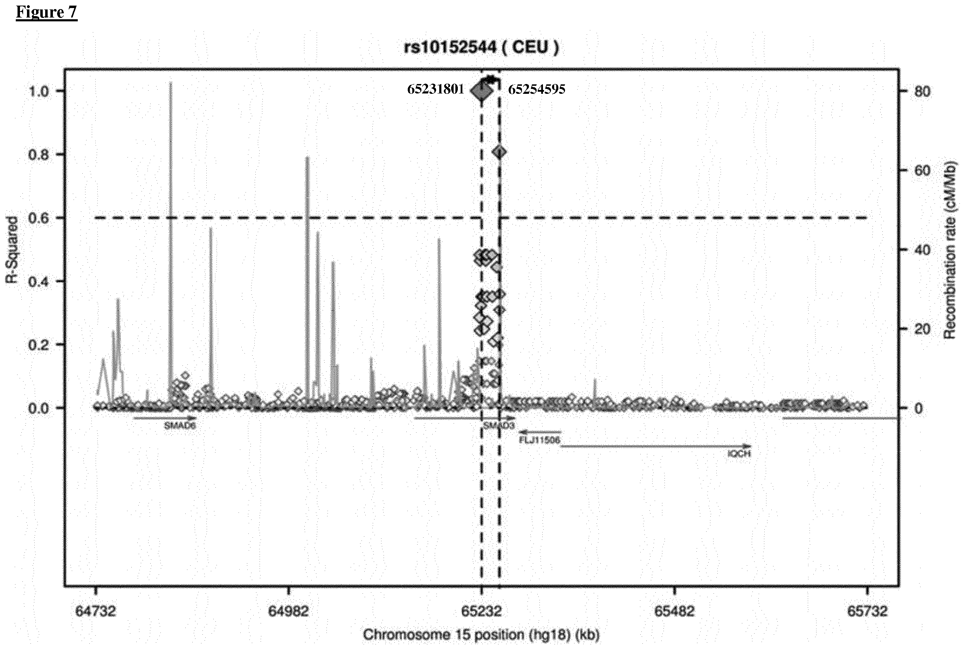

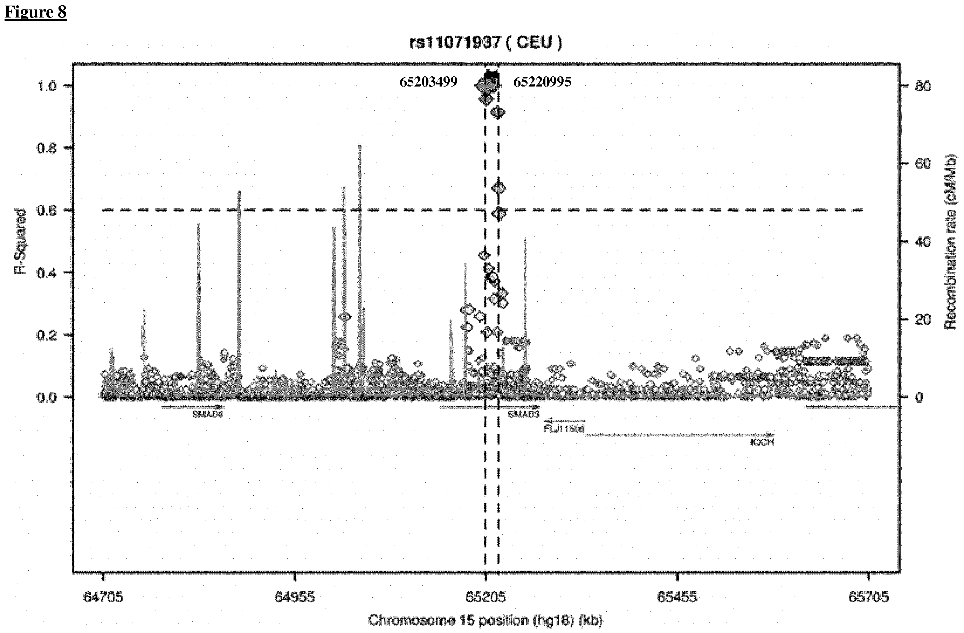

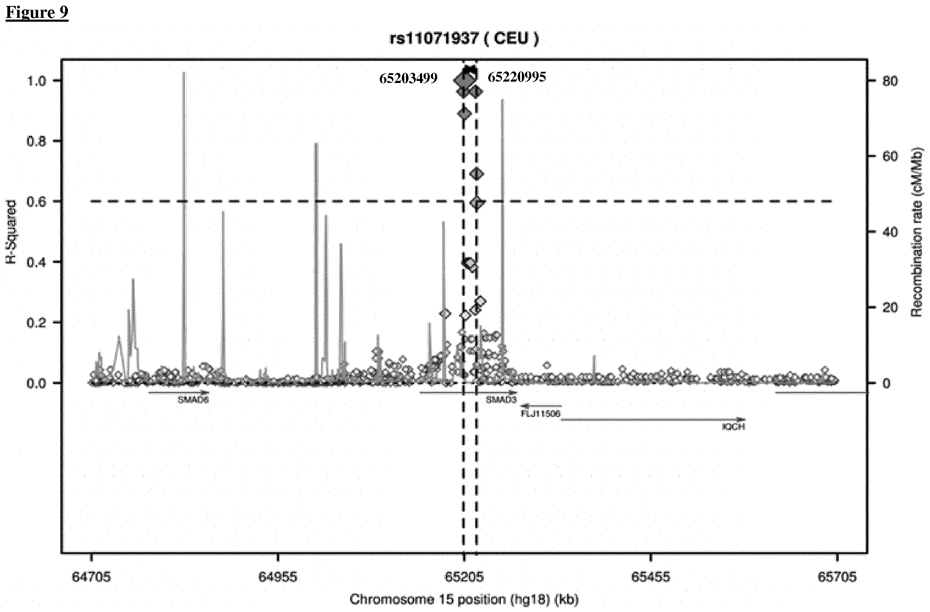

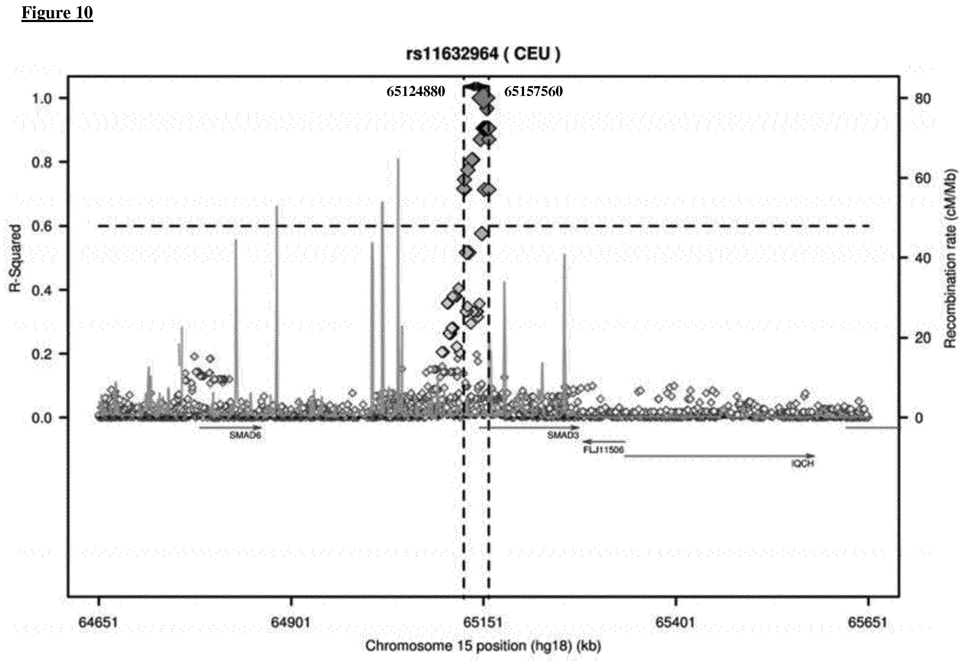

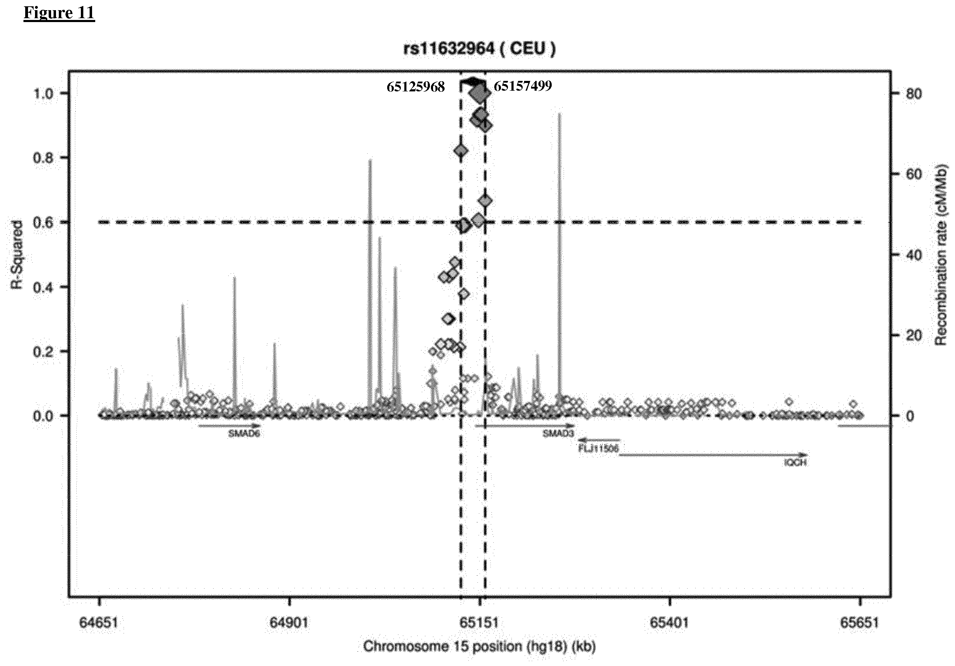

Thus, SNPs rs11071937 and rs11632964 in SMAD3, or other variants highly correlated with either of these SNPs, modulate susceptibility to HF. The inventors built a map of all SNPs correlated (due to linkage disequilibrium) with either SNP rs11071937, or SNP rs11632964 in a region extending 500 kb from the 3' and 5' end of *SMAD3* to rule out the possibility that the casual variants may lie outside of *SMAD3.* Figures 5-11 show the various common SNPs (MAF>5%) in the regions around SNPs rs11071937 and rs11632964 and their correlation with the SNPs of interest (Y axis). Data were obtained either from 1000 Genome's or HAPMAP's projects data bank. The inventors have also included the same data on SNPs rs1470001 and rs10152544, since these SNPs will be discussed when SNPs from SMAD3 and BMP7 will be tested in the same model.

None of these SNPs outside *SMAD3* were strongly correlated with SNPs rs11071937, rs1470001 and rs10152544 and could account for the association. Then, the causal SNP which is in LD with SNPs rs11071937, rs1470001 and rs10152544 must lie in SMAD3.

There are, however, few SNPs lying outside SMAD3 that correlate enough with SNP rs11632964 to possibly account for the association. These are SNPS rs3101491, rs347222686, rs7181798, rs6494626, rs6494624 and rs6494627, which are located within 8 to 19.3 Kb of the 3' end of SMAD3. There is no other gene in that region reported so far. Then, the inventors cannot formally exclude that the causal SNP which is uncovered by the association of HF with SNP rs11632964, lies within this region outside SMAD3 possibly in a regulatory sequence.

Interestingly SNPs rs1470001 and rs10152544 that the inventors show below to be associated with HF in a model with BMP7, are not correlated (r²>0.6) with SNPs outside SMAD3, further supporting the conclusion that at least 3 (and likely four) causal SNPs located in SMAD3 regulate hepatic fibrosis.

### Three SNPs in BMP7 are independently associated with severe HF

The inventors have selected 30 tag-SNPs in BMP7 and genotyped them in the cohort by the Sequenom method. The inventors replicated the genotyping using a TaqMan man assay with less than 2% errors for 27 SNPs. 3 SNPs were not well replicated and were not included in the analysis. The inventors have obtained significant (p≤0.01) associations with 6 SNPs in BMP-7. The inventors present in Table 2 the data obtained with the 3 SNPs that showed independent associations in the multivariate analysis.

**Table 2. BM7 genotypes associated with advanced hepatic fibrosis in subjects infected with S. mansoni**

| **SNP** | **Aggravating genotypes** | **% in controls¹** | **% in cases¹** | **p** | **OR** | **CI** | **r²** |
|---|---|---|---|---|---|---|---|
| **Univariate analysis** | | | | | | | |
| rs162314 | CC,TT | 55.7 | 65.5 | 9.10⁻³ | 1.53 | (1.1 - 2.1) | 0.014 |
| rs6127988 | TT,CT | 29.3 | 40.5 | 10⁻³ | 1.72 | (1.2 - 2.4) | 0.022 |
| rs7352741 | CC | 41.6 | 52.9 | 4.10⁻³ | 1.60 | (1.16-2.16) | 0.018 |

| **Multivariate analysis** | | | | | | | |
|---|---|---|---|---|---|---|---|
| rs162314 | CC,TT | | | 8.10⁻³ | 1.56 | (1.12-2.22) | |
| rs6127988 | TT,CT | | | 0.01 | 1.56 | (1.1 - 2.2) | 0.048 |
| rs7352741 | CC | | | 5.10⁻³ | 1.5 | (1.1 - 2.20) | |

| | | | | | | | |
|---|---|---|---|---|---|---|---|
| ¹ Percentage of subjects with the aggravating genotypes in 349 Controls and 307 Cases. Controls are subjects who have been exposed to infections for > 20 years and show no (grade A) or mild (grade B or C) hepatic fibrosis. Cases are subjects with grade E or F Hepatic Fibrosis r² = (r Nagelkerke)² | | | | | | | |

The SNPs rs162314 (9x10⁻³, 1.5), rs6127988 (10⁻³, 1.7) and rs7352741 (4x10⁻³, 1.6) showed significant associations with HF in the univariate analysis with r² varying from 0.014 to 0.022. These same SNPs, rs162314 (8x10⁻³, 1.56), rs6127988 (10⁻³, 1.56) and rs7352741 (5x10⁻³, 1.5), remained associated in the multivariate analysis and the regression model accounted for 4.8% of phenotypic variance.

### The causal SNPs in LD with SNPs rs162314, rs6127988 and rs7352741 lie in BMP7

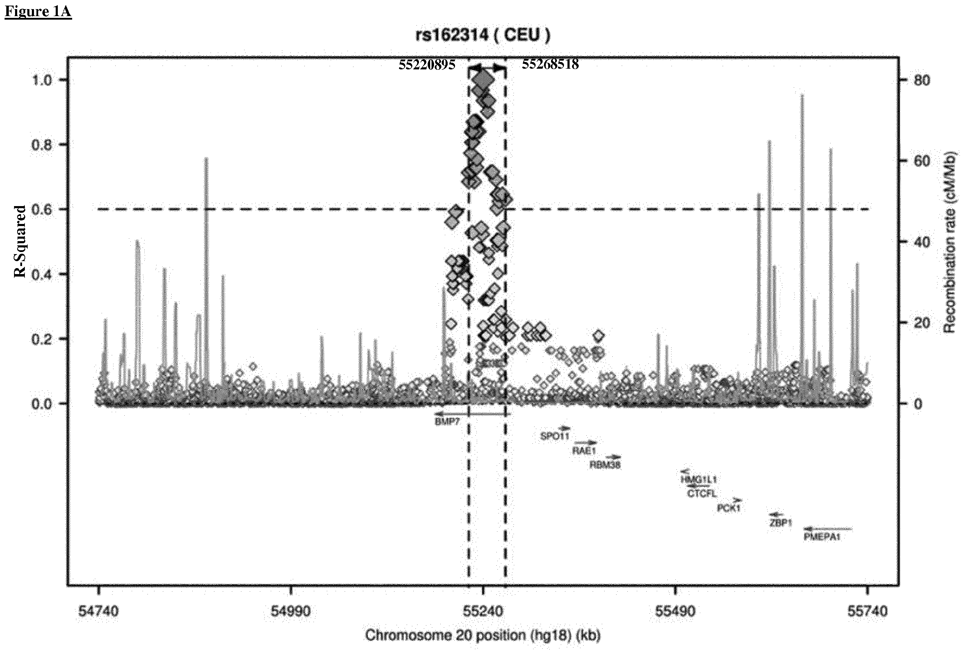

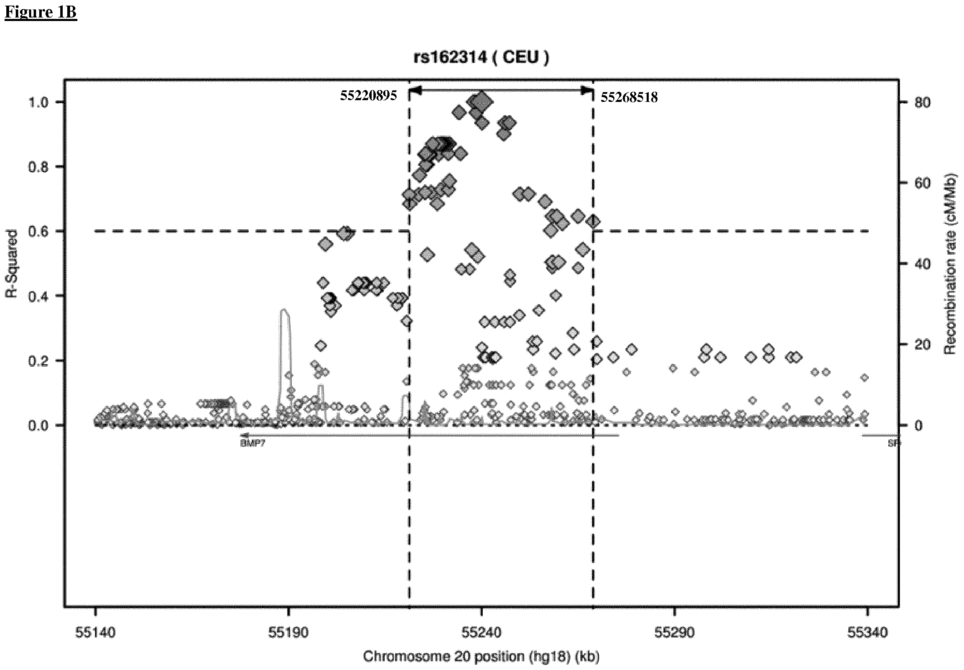

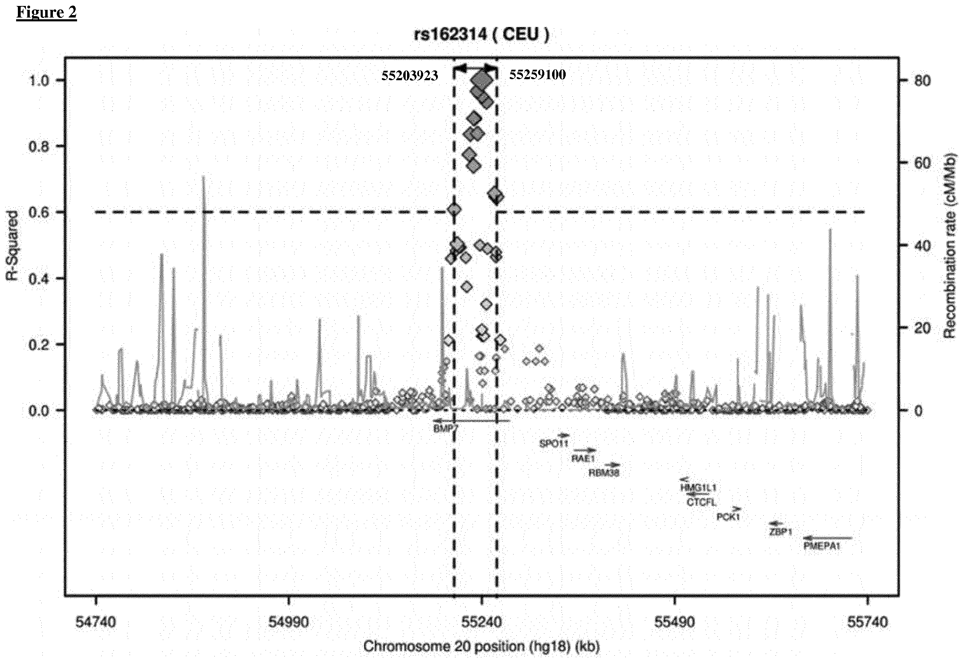

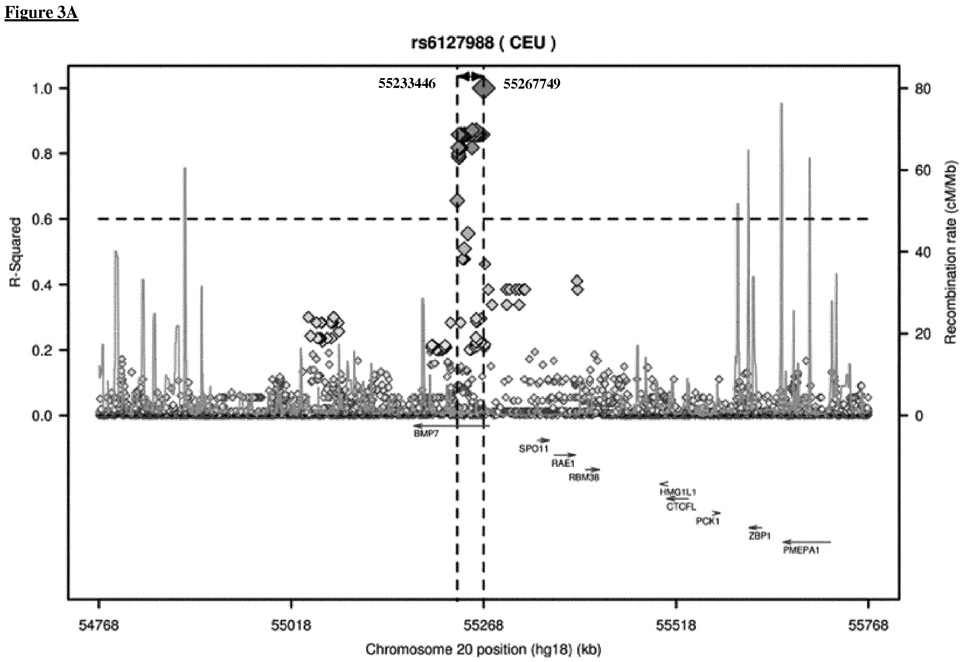

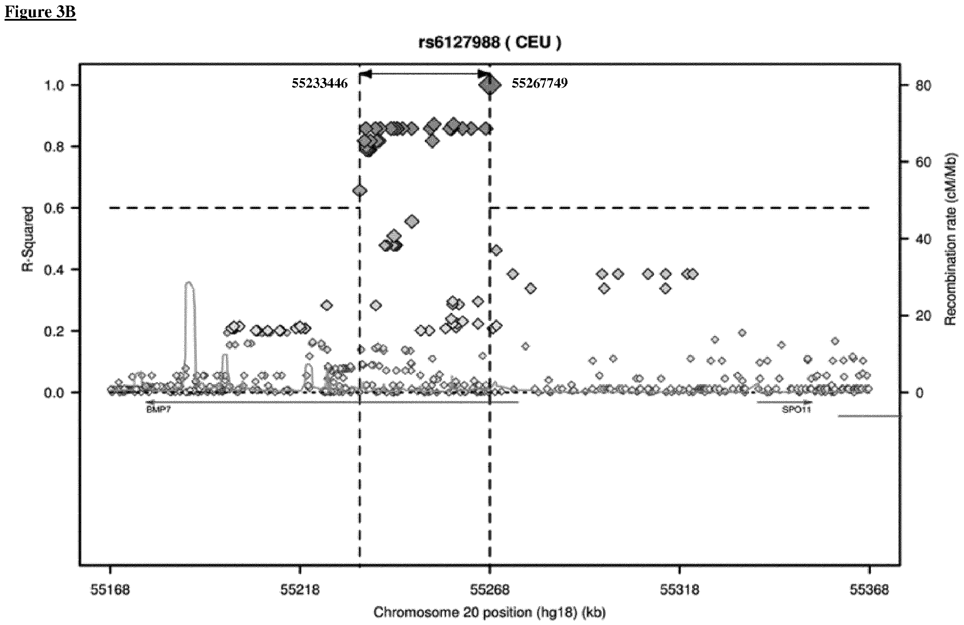

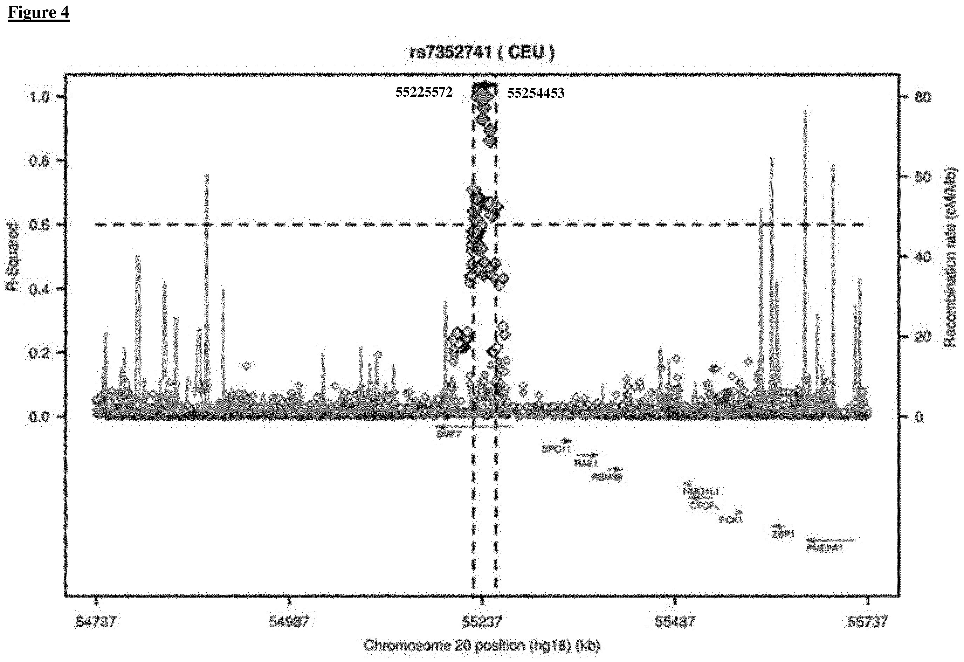

These data show that SNPs rs162314, rs6127988 and rs7352741 in BMP7, or other variants highly correlated with either of these SNPs, alter susceptibility to HF. The inventors built a map of all SNPs correlated with SNP rs162314, SNP rs6127988 or SNP rs7352741 in a region extending 5 Mb 500 kb from the 3' and 5' end of *BMP7* to rule out the possibility that the casual variants may lie outside of *BMP7.* Data were obtained either from 1000 Genome's or HAPMAP's projects data bank. Figures 1-4 show the various common SNPs (MAF>5%) in this region and their correlation with the SNPs of interest (Y axis). None of these SNPs outside *BMP7* were strongly correlated with SNPs rs162314, rs6127988 and rs7352741 and could account for the association. Then, the causal SNPs lie in *BMP7.*

### Simultaneous testing of the genetic variants in SMAD3 and BMP7 markedly improves the regression model

Since BMP7 and TGF-β pathways are competing for the recruitment of Smad4 down of their receptor to allow translocation of oligomeric R-Smads complex to the nucleus, the inventors made the hypothesis that testing SMAD3 and BMP7 polymorphisms in the same model would improve the associations by accounting the competition for Smad4 between the two pathways. The data of this multivariate regression analysis are shown in Table 3.

**Table 3. Mudtivariate regression analysis of the SMAD3 and BMP7 genotypes associated with advanced hepatic fibrosis**

| **GENE** | **SNP** | **Aggravating Genotypes** | **p** | **OR** | **CI** |
|---|---|---|---|---|---|
| SMAD3 | rs1470001 | TT | 0.01 | 1.6 | (1.1 - 2.27) |
| SMAD3 | rs10152544 | CC,CT | 0.02 | 1.7 | (1.07 - 2.7) |
| SMAD3 | rs11071937 | GT | 2.10⁻⁴ | 1.9 | (1.4 - 2.7) |
| SMAD3 | rs11632964 | CC | 5.10⁻⁴ | 1.8 | (1.3 - 2.5) |
| BMP7 | rs162314 | CC,TT | 9.10⁻⁴ | 1.82 | (1.3 - 2.6) |
| BMP7 | rs6127988 | TT+CT | 0.02 | 1.5 | (1.4 - 2.1) |
| BMP7 | rs7352741 | CC | 5.10⁻⁴ | 1.64 | (1.16 - 2.3) |

| | | | | | |
|---|---|---|---|---|---|
| Same legend as in Tables 1 and 2. r²= 0.12 | | | | | |

Strikingly, the simultaneous testing of SNPs from both genes increase the strength of the association of SMAD3 rs1470001 and rs10152544 allowing them to enter the regression model. This analysis also improved SNP rs11071937 and rs11632964 associations, increasing the p values of the associations and the OR associated with these SNPs. In this regression model that included SNPs from both SMAD3 and BMP7, the association with all SNPs but BMP7 rs6127988 showed higher OR and lower p (10 fold lower for most p values) than in the multivariate regression models that accommodated SNPs from either two genes (lower parts of tables 1 and 2). Most important this two gene regression model accounted for 12% of phenotypic variance as compared to 8.6 % (4.6% BMP7 + 4% SMAD3 model under the assumption of additive variance) when the two genes were tested separately.

Then, detecting the SNPs of interest in SMAD3 and BMP7 genes as explained in this study allows a reliable and sensitive detection of aggravating genotypes correlated with fibrosis, and thus, a better prediction of fibrosis progression.

Thus, the method according to the present invention allows predicting if a subject would develop a severe fibrosis, or would be already afflicted by a severe fibrosis.

## Claims

1. Method for detecting a predisposition to and/or prognosing fibrosis progression of a subject in need thereof, comprising detecting at least one single nucleotide polymorphism (SNP) in SMAD3 gene and at least one SNP in BMP7 gene, in a biological sample of said subject, wherein the presence of at least one SNP in SMAD3 gene and at least one SNP in BMP7 gene are indicative of a predisposition to fibrosis or of fibrosis progression.

2. Method according to claim 1, wherein said fibrosis is chosen from:
- hepatic fibrosis, comprising hepatic fibrosis of various grades including cirrhosis, alcoholic and non-alcoholic,
- fibrosis of the skin tissue, particularly due to cutaneous keloid, hypertrophic scars or scleroderma,
- fibrosis of the adipose tissue, such as fibrosis due to obesity, such as non-alcoholic fatty liver disease or non-alcoholic steatohepatitis,
- pulmonary fibrosis, and
- kidney fibrosis.

3. Method according to claim 1 or 2, wherein said fibrosis is hepatic fibrosis, particularly due to a chemical agent, such as an hepato-toxic drug or alcohol; due to fat, such as non-alcoholic fatty liver disease or non-alcoholic steatohepatitis; due to a bacterial infection, such as brucellosis; due to a parasitic infection, such as bilharzia; or due to viral infections, such as hepatic A virus, hepatic B virus or hepatic C virus infections.

4. Method according to any one of claims 1 to 3, wherein said biological sample is a biopsy, a blood sample, a saliva sample or a urine sample.

5. Method according to any one of claims 1 to 4, wherein the subject is infected with a virus, preferably a virus selected from the Flaviviridae, such as Hepatitis A or C or B Virus; or the subject is infected by Schistosoma, such as *Schistosoma mansoni, Schistosoma haematobium, Schistosoma japonicum, Schistosoma mekongi* or *Schistosoma intercadatum;* or the subject has a diet which is fat-rich, and/or alcohol-rich, preferably is afflicted by fatty liver, overweight, obesity and/or alcoholism.

6. Method according to any one of claims 1 to 5, wherein the SNP in SMAD3 gene is chosen from rs1470001, rs10152544, rs11071937 and rs11632964.

7. Method according to any one of claims 1 to 6, wherein the SNP in BMP7 gene is chosen from rs162314, rs6127988 and rs7352741.

8. Method according to any one of claims 1 to 7, wherein it comprises detecting, in a biological sample of said subject:
a) the presence of at least one of the following genotypes in SMAD3 gene locus:
- genotype TT with respect to SNP rs1470001,
- genotype CC or CT with respect to SNP rs 10152544,
- genotype GT with respect to SNP rs11071937, and
- genotype CC with respect to SNP rs11632964, and
b) the presence of at least one of the following genotypes in BMP7 gene locus:
- genotype CC or TT with respect to SNP rs162314,
- genotype TT or CT with respect to SNP rs6127988, and
- genotype CC with respect to SNP rs7352741,
wherein a) the presence of at least one SNP in SMAD3 gene locus and b) the presence of at least one SNP in BMP7 gene locus are indicative of a predisposition to fibrosis or of fibrosis progression.

9. Kit for detecting a predisposition to and/or prognosing fibrosis progression, comprising at least the sequences SEQ ID NO:8 to SEQ ID NO:14.


SEQUENCE LISTING

<110>  GENEPRED BIOTECHNOLOGIES
       INSERM
       UNIVERSITE AIX MARSEILLE

<120>  Method for prognosing fibrosis progression

<130>  BEP150946EP

<160>  14

<170>  PatentIn version 3.5

<210>  1
<211>  20
<212>  DNA
<213>  Artificial Sequence

<220>
<223>  y is C or T

<400>  1
ggaacatgay atggtctatg                                                   20


<210>  2
<211>  19
<212>  DNA
<213>  Artificial Sequence

<220>
<223>  y is C or T

<400>  2
gctgccgagy gagaggccc                                                    19


<210>  3
<211>  20
<212>  DNA
<213>  Artificial Sequence

<220>
<223>  k is G or T

<400>  3
tgatccaccc kcttctttct                                                   20


<210>  4
<211>  11
<212>  DNA
<213>  Artificial Sequence

<220>
<223>  y is C or T

<400>  4
gactgyagta t                                                            11


<210>  5

<211>  20
<212>  DNA
<213>  Artificial Sequence

<220>
<223>  y is C or T

<400>  5
tatcatcttt ygtatctggc                                                    20


<210>  6
<211>  19
<212>  DNA
<213>  Artificial Sequence

<220>
<223>  y is C or T

<400>  6
aagcatcagy gagggctgg                                                     19


<210>  7
<211>  20
<212>  DNA
<213>  Artificial Sequence

<220>
<223>  y is C or T

<400>  7
atacacctty tttagaaggg                                                    20


<210>  8
<211>  51
<212>  DNA
<213>  Artificial Sequence

<220>
<223>  y is C or T

<400>  8
tagacaagac aaaaagggaa catgayatgg tctatggctc tggttggtgg a                 51


<210>  9
<211>  51
<212>  DNA
<213>  Artificial Sequence

<220>
<223>  y is C or T

<400>  9
ctacatgaac aggcctgctg ccgagygaga ggcccagatc acccagaatg a                 51


<210>  10
<211>  51
<212>  DNA
<213>  Artificial Sequence

<220>
<223>  k is G or T

<400>  10
ggcagctctg gttgctgatc caccckcttc tttctttata gagattaaga g          51


<210>  11
<211>  51
<212>  DNA
<213>  Artificial Sequence

<220>
<223>  y is C or T

<400>  11
tatccaaagg aagctttttg gactgyagta tgatgttggg aactttttgg t          51


<210>  12
<211>  51
<212>  DNA
<213>  Artificial Sequence

<220>
<223>  y is C or T

<400>  12
gggcctcata cactatatca tctttygtat ctggcttctt ccatttcaca g          51


<210>  13
<211>  51
<212>  DNA
<213>  Artificial Sequence

<220>
<223>  y is C or T

<400>  13
ttggccaggc agccccaagc atcagygagg gctggaagcc ttcactgtga a          51


<210>  14
<211>  51
<212>  DNA
<213>  Artificial Sequence

<220>
<223>  y is C or T

<400>  14
ggaagtgaca tacaccatac accttyttta gaagggataa tctggtacag g          51